# EUROPÄISCHE PATENTSCHRIFT

(11) **EP 1 653 845 B1**
(45) Veröffentlichungstag und Bekanntmachung des Hinweises auf die Patenterteilung: **03.12.2008**
(21) Anmeldenummer: 04762483.8
(22) Anmeldetag: 22.07.2004
(51) Int. Cl.: A61B 1/018

(54) **FLEXIBLES ENDOSKOP MIT LÄNGSAXIALEN KANÄLEN**
FLEXIBLE ENDOSCOPE WITH LONGITUDINAL AXIAL CHANNELS
ENDOSCOPE FLEXIBLE COMPORTANT DES CANAUX AXIAUX LONGITUDINAUX

(30) Priorität: 25.07.2003 DE 10334100
(43) Veröffentlichungstag der Anmeldung: 10.05.2006
(73) Patentinhaber: Karl Storz GmbH & Co. KG, 78532 Tuttlingen (DE)
(72) Erfinder: WIMMER, Viktor Josef, 83370 Seeon (DE)
(74) Vertreter: Mischung, Ralf
(86) Internationale Anmeldenummer: PCT/DE2004/001633
(87) Internationale Veröffentlichungsnummer: WO 2005/011482

(56) Entgegenhaltungen:
- EP-A- 0 049 851
- WO-A-00/48505
- DE-A- 2 429 476
- US-A- 5 924 976

## Beschreibung

Die vorliegende Erfindung betrifft ein flexibles Endoskop mit längsaxialen Kanälen nach dem Oberbegriff des Anspruchs 1.

Der Einsatz von Endoskopen ist aus der Medizintechnik bekannt. Die im Wesentlichen entlang einer Längsrichtung ausgebildeten Endoskope werden in den menschlichen Körper eingeführt, um dort operative Eingriffe vorzunehmen. Koloskope sind Endoskope, die im Darmbereich eingesetzt werden. Die Endoskope verfügen dabei nach dem Stand der Technik über geeignete Beleuchtungs- und Bildaufnahmeelemente, um die zu behandelnde Stelle im Körper optisch erfassen und kontrollieren zu können. Weiterhin weisen die bekannten Endoskope Kanäle auf, welche zur Spülung oder auch zur Durchführung von Werkzeugen verwendet werden. Insbesondere bei den einfachen Endoskopen erstrecken sich die Kanäle bis zum distalen Ende des Endoskops, welches üblicherweise als Kopf bezeichnet wird. Dort treten die Kanäle bevorzugt stirnseitig, also in Längsrichtung des Endoskops, aus, so dass insbesondere ein in Längsrichtung durch einen Arbeitskanal geschobenes Werkzeug stirnseitig aus dem Kopf herausgeschoben werden kann.

Ein Arbeitskanal, durch den ein Werkzeug hindurchgeschoben werden kann, gibt diesem eine geeignete Führung, um es einigermaßen gezielt aus dem Kopf herausschieben und dort einsetzen zu können. Nachteilig ist bei den solchermaßen bekannten Endoskopen, dass durch die gezielte Führung auch nur ein eingeschränkter Bereich jenseits der Stirnseite des Endoskopkopfes durch das Werkzeug erreicht werden kann. Insbesondere ist es schwierig, das Werkzeug in einen seitlich der Längsachse liegenden Bereich zu bewegen. Zwar kann in einer gewissen Entfernung von einer zu bearbeitenden Stelle (bspw. ein Polyp an der Innenwand des Darms) diese Stelle durch die "nach vorne blickende" Optikeinheit erfasst werden. Das Werkzeug kann jedoch aus der Längsrichtung des Endoskops nicht oder nur unpräzise an die Darmwand bewegt werden. Der optisch erfasste und der zu bearbeitende Bereich sind oftmals nicht identisch. Der Einsatzbereich ist damit eingeschränkt.

Aus der DE 2429476 ist ein Endoskop mit einem Durchlass in einer Stirnfläche am distalen Ende bekannt, durch den ein Werkzeug hindurchbewegbar ist. Das Werkzeug ist dabei von einem mittels Betätigungsdraht bedienbaren Steuerglied im wesentlichen zwischen zwei Lagen schwenkbar.

Die US 5,924,976 beschreibt ein Operationsinstrument nach Art eine Endoskops, bei dem mehrere im Wesentlichen kugelförmige Elemente zur Führung verschiedener Werkzeuge dienen, welche durch die distale Stirnseite hindurchschiebbar sind.

Aufgabe der Erfindung ist, ein Endoskop zu schaffen, mit dem verschiedene Werkzeuge räumlich variabler und gleichzeitig gut geführt einsetzbar sind.

Die Aufgabe wird gelöst durch ein Endoskop nach Anspruch 1.

Die Erfindung geht von der Erkenntnis aus, dass das Werkzeug räumlich flexibler verwendet und gleichzeitig gut geführt werden kann, wenn ein Teil des das Werkzeug führenden Arbeitskanals im Bereich des Endoskopkopfs gelenkig ausgebildet wird. Insbesondere soll das gelenkige Teil (welches im Weiteren als Führungselement bezeichnet wird) um eine Schwenkachse schwenkbar sein, die im Wesentlichen senkrecht zur Längsrichtung des Endoskops verläuft.

Erfindungsgemäß verläuft der Arbeitskanal für das oder die Werkzeuge zunächst entlang der Längsrichtung des Endoskops. Im Kopfbereich des Endoskops durchläuft der Arbeitskanal, bevor er an der Stirnseite des Endoskopkopfs austritt, das Führungselement. Im unverschwenkten Zustand ist der letzte Abschnitt des Arbeitskanals im Wesentlichen fluchtend mit dem zum proximalen Ende des Endoskops führenden Arbeitskanal ausgerichtet. Das distale Ende des Führungselements fällt damit mit einer gedachten, den Endoskopkopf distal abschließenden Stirnseite zusammen. Ein durch den Arbeitskanal des Endoskops und auch den Arbeitskanal des Führungselements durchgeschobenes Werkzeug tritt dann an der Stirnseite des Kopfes aus, wobei es sich im Wesentlichen parallel zur Längsrichtung des Endoskops aus dem Arbeitskanal heraus bewegen bzw. hineinschieben lässt.

Durch die erfindungsgemäß vorgesehene Möglichkeit des Verschwenkens des Führungselements kann nun das Werkzeug von der Längsrichtung des Endoskops abgewinkelt werden, so dass auch ein seitlicher, aus der Stirnseite heraustretender, in Längsrichtung liegender Bereich von dem Werkzeug bearbeitet werden kann. Das Endoskop ist also vorteilhafterweise nicht nur darauf beschränkt, im Wesentlichen in Längsrichtung des Endoskops herausgeschoben zu werden, um in dieser Richtung zu arbeiten. Vielmehr kann es zusätzlich durch Verschwenken des Führungselements auch einen im Wesentlichen schräg vor dem Endoskopkopf liegenden Bereich erfassen.

Ein Verschwenken soll dabei erfindungsgemäß auf eine oder beide Seiten einer gedachten Ebene möglich sein, die sich entlang der Achse des Arbeitskanals des Endoskops erstreckt. In einer verschwenkten Anordnung bildet die Längsachse des Arbeitskanals im Endoskop mit der Längsachse des Arbeitskanals im Führungselement dann einen Winkel α ≠ 180°.

Dies erhöht den Einsatzbereich beträchtlich, da das Werkzeug Zugang zu einem weiteren Bereich jenseits der Stirnseite des Endoskops und insbesondere seitlich zu dessen Längsachse hat. Durch die Möglichkeit des Schwenkens des Arbeitskanals sind Optikeinheit und Arbeitskanal bzw. Instrumentenkanal separat steuerbar, so dass eine Relativbewegung zwischen der Optikeinheit und dem im Führungselement ausgebildeten letzten Abschnitt des Arbeitskanal möglich ist. Dadurch kann der von der optischen Einheit erfasste Bereich und der vom Werkzeug erreichbare Bereich vorteilhaft aufeinander abgestimmt werden.

Durch die Ausbildung des letzten Abschnittes des Arbeitskanals als Führung wird eine sehr genaue Handhabung des durch den Kanal geschobenen Werkzeugs möglich. Da diese Führung das Werkzeug oder dessen Schaft allseitig umschließt, kann es in seitlicher Richtung gedrückt und aus dieser Lage auch wieder aktiv herausgeschwenkt werden. So ist gegenüber den aus dem Stand der Technik bekannten Albarranhebeln auch eine Zugbewegung aus der seitlichen (ausgelenkten) Position des Werkzeugs zurück in Richtung auf die zentrale Längsachse des Endoskops möglich.

Eine vorteilhafte Ausführungsform der Erfindung sieht vor, das Führungselement an seinem proximalen Ende mit geeigneten Einführhilfen zu versehen, die das Durchschieben eines Werkzeugs erleichtern. So kann, anders als beim Albaranhebel, insbesondere auch im abgewinkelten (ausgeschwenkten) Zustand des Führungselements vom proximalen Ende des Endoskops her ein Werkzeug durch den Arbeitskanal geschoben werden, welches mittels der Einführhilfen in das Führungselement weitergefädelt wird. Eine einfache Einführhilfe kann bspw. eine trichterförmige Mulde am proximalen Ende des Führungselements sein.

Eine besonders einfache Ausführungsform der Erfindung sieht vor, dass die Schwenkachse, um die das Führungselement schwenkbar ist, im Wesentlichen quer zur Längsrichtung des Endoskops angeordnet ist. Dies ermöglicht vorteilhaft die maximale Auslenkung bei geringem Schwenkwinkel. Je geringer der Schwenkwinkel, umso geringer fällt die erforderliche Manipulation am proximalen Ende des Endoskops aus. In dem vereinfachten konstruktiven Aufbau wird damit auch die Handhabung erleichtert.

Eine weitere vorteilhafte Ausführungsform der Erfindung sieht vor, dass sich neben dem schwenkbaren Führungselement ein starrer Abschnitt des Kopfs bis zur Stirnseite erstreckt. Ein solcher Bereich kann vorteilhaft weitere Funktionen des Endoskops ermöglichen und beispielsweise zur Aufnahme von Arbeitskanälen und/oder optischen Einheiten und/oder Medienkanälen und/oder als Anschlag für das Führungselement (F) ausgebildet sein. Die Medienkanäle dienen vorteilhaft zur Spülung oder Absaugung.

Solche Kanäle können stabil und gut geschützt bis zur Stirnseite des Kopfes geführt werden, wo sie austreten (Spülungskanäle) oder durch ein Linsensystem abgeschlossen werden (optisches System). Als weiterer Vorteil eines solchen starren Abschnitts ergibt sich, dass das gelenkige Führungselement gegen seitliche Kräfte geschützt wird, die von dem starren Abschnitt aufgenommen werden können. Dies macht das Führungselement und damit den Endoskopkopf unempfindlicher gegen ungewollte mechanische Einflüsse und erhöht damit dessen Lebensdauer.

Die Erfindung sieht vor, dass zumindest der nicht gelenkige Teil des Arbeitskanals gleichzeitig als Spül- oder Saugkanal ausgebildet ist. So kann er gleichzeitig die Führung des Werkzeugs und die Röhrenform des Kanals nutzen, um in einem Kanal zwei Funktionen platzsparend zu kombinieren.

Die Erfindung sieht weiterhin vor, dass die Schwenkbewegung des Führungselements durch wenigstens einen Anschlag in wenigstens einer Schwenkrichtung begrenzt wird. Ein solcher Anschlag stabilisiert das Führungselement und verhindert ein unkontrolliertes Bewegen desselben. Insbesondere kann der Anschlag auch als Anlage gegen eine federvorgespannte Zwangsbewegung dienen, wenn nämlich das Führungselement gegen eine Feder aus der Anschlagsposition oder einer anderen vorgebbaren Lage herausbewegt werden kann. Bei Entlastung des das Führungselement bewegenden Betätigungszuges (Bowdenzug oder sonstiges Bedienelement) legt sich dieses vorteilhaft gegen den Anschlag an, und kommt so in eine definierte Ruheposition. Die Operation wird dadurch sicherer und übersichtlicher.

Der Bowdenzug kann dabei gegen eine Druckfeder und/oder eine Zugfeder oder ein sonstiges geeignetes Rückstellelement arbeiten, wobei dieses Element im Bereich des Bowdenzugs oder auch an anderer Stelle angeordnet sein kann, um eine Relativbewegung zwischen Schwenkelement und Kopf zu erzwingen.

Erfindungsgemäß ist das Betätigungselement, wie etwa ein Bowdenzug, in einem Kanal geführt und aus diesem zu Reinigungs- oder Wartungsarbeiten entnehmbar. Auch das Führungselement ist besonders vorteilhaft lösbar angeordnet, um eine Reinigung oder Wartung des Endoskops besser vornehmen zu können.

Ebenfalls vorteilhaft ausgebildet ist eine Ausführungsform der Erfindung, bei der der Arbeitskanal in seinem distalen (End-)Abschnitt im Führungselement einen geringeren Durchmesser aufweist als in seinem proximalen ("unbewegten") Abschnitt. Da der proximale Abschnitt des Arbeitskanals zugleich als Spül- oder Saugleitung verwendet werden kann, ist ein entsprechend vergrößerter (Ring-)Spalt zwischen Kanalwand und im Kanal geführtem Werkzeug vorzusehen. Hingegen soll der distale ("bewegte") Abschnitt des Arbeitskanals im Führungselement vorwiegend zur genauen Führung des Werkzeuges dienen und dieses daher möglichst knapp umschließen. Zur Beibehalten einer guten Saugwirkung ist das Führungselement daher mit Ausnehmungen, insbesondere länglichen Vertiefungen in im Wesentlichen axialer Richtung versehen. Diese Vertiefungen auf der Innenseite (in der Innenwand des Arbeitskanals des Führungselements) und/oder auf der äußeren Seite des Führungselements, erlauben eine verbesserte Saugleistung bis zum distalen Ende des Endoskops, die durch eine den Kopf umgebende Hülse noch erhöht wird. Die Saugwirkung kann dabei durch den Arbeitskanal im Führungselement und um das Führungselement herum/daran vorbei aufrecht erhalten werden.

In vorteilhafter Weise kann der Anschlag auch durch den starren Abschnitt ausgebildet werden, der sich seitlich zum Führungselement bis zur Stirnseite erstreckt. Auch hier kann das Führungselement im unbelasteten Zustand seitlich an dem starren Abschnitt ruhen, so dass es innerhalb und außerhalb des menschlichen Körpers eine gegenüber der ausgeschwenkten Lage gestütztere Position einnimmt.

Weitere vorteilhafte Ausführungsformen der Erfindung betreffen die Anordnung der Schwenkachse des Führungselements. Definiert man für den letzten Abschnitt des Arbeitskanals im Führungselement eine Arbeitskanalachse B' und im davor liegenden sonstigen Bereich des Arbeitskanals eine Arbeitskanalsachse B, so fluchten die beiden Achsen B und B' im nichtausgelenkten Zustand des Führungselements. Der Arbeitskanal erstreckt sich damit durch den Endoskopkopf unabgewinkelt hindurch. Im abgewinkelten Zustand dagegen, schließen die beiden Achsen B und B' einen Winkel α ≠ 180° ein.

In einer vorteilhaften Ausführungsform der Erfindung ist die Schwenkachse des Führungselements in dem vom Winkel α eingeschlossenen Bereich angeordnet. Insbesondere aus der unten angeführten Figurenbeschreibung ist ersichtlich, dass in diesem Fall die Schwenkachse im äußeren Bereich des Endoskopkopfs angeordnet sein kann. Der Vorteil dieser Ausführungsform liegt darin, dass das Führungselement konstruktiv einfach beschaffen ist und eine größtmögliche Führung für das durchgeschobene Werkzeug bieten kann. Des Weiteren ist die Schwenkachse besser zugängig, so dass das Führungselement leichter zu entnehmen, zu reinigen oder zu warten ist.

In einer anderen vorteilhaften Ausführungsform der Erfindung ist die Schwenkachse in dem vom Winkel α ausgeschlossenen Bereich angeordnet. In diesem Verlauf verläuft die Schwenkachse vorzugsweise im Inneren des Endoskopkopfs, was den Vorteil eines besseren Schutzes vor Verschmutzung bietet. Weiterhin ermöglicht dieser Fall eine konstruktive Ausbildung des Führungselements mit geringerem Materialaufwand. Des Weiteren ergibt sich der Vorteil, dass im unausgeschwenkten Zustand zwischen dem Führungselement und dem restlichen Kopf ein Öffnungswinkel entsteht, durch den insbesondere Reinigungsvorgänge des Arbeitskanals bzw. des im Führungselement liegenden letzten Abschnitts des Arbeitskanals ermöglicht werden. Auch ermöglicht insbesondere die zentrale Anordnung der Schwenkachse ein symmetrisches Verschwenken auf beide Seiten des Kopfes und erlaubt damit zumindest teilweise symmetrische Fertigungsgeometrien.

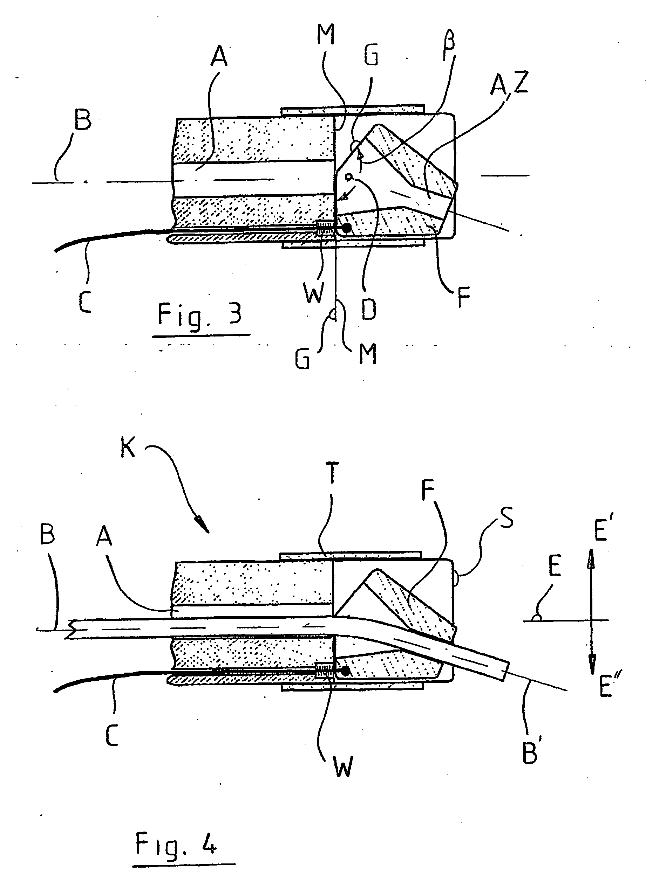

Eine weitere vorteilhafte Ausführungsform der Erfindung sieht vor, das die proximale Begrenzung des Führungselements zwei einen Winkel β < 180° bildende Stirnflächen aufweist, in deren gedachtem Schnittbereich die Schwenkachse so angeordnet ist, dass die Stirnflächen als die Schwenkbewegung begrenzende, mit Anschlagabschnitten des Kopfes zusammenwirkende Anschlagflächen ausgebildet sind. Eine solche Form ermöglicht ein beidseitiges "Kippen" des Führungselementes relativ zu einer gedachten, das Endoskop mittig teilenden Ebene, jeweils bis zu einem Anschlag, den die Stirnflächen des Führungselementes mit Flächen des Kopfes bildet, wie dies später insbesondere aus den Figuren 3 und 4 zu sehen sein wird. Der Vorteil liegt in einer einfachen Begrenzung des Schwenkwinkels, wobei durch die winklige proximale Stimseitenausbildung des Führungselements eine Schwenkbewegung auf beide Seiten der gedachten Ebene möglich ist.

Je nach Form und Ausbildung der Stirnflächen und der damit zusammenwirkenden Anschlagflächen des Kopfes wird dabei eine zur Ebene asymmetrisch oder auch asymmetrisch erfolgende Schwenkbewegung möglich. Die Anschlagflächen des Kopfes können am distalen Ende des "unbewegten" Arbeitskanals oder auch über einen in radialer Richtung wirkenden Anschlag realisiert sein.

Das Endoskop weist eine sich in Längsrichtung erstreckende, den Kopf radial umschließende und in distaler Richtung offene Hülse auf. Diese Hülse dient zur Vergößerung einer Absaugfläche, die an der Stirnseite eines Absaugkanals üblicherweise entsteht. Die Hülse ist in proximaler Richtung mit bzw. durch den Kopf gegen Fehlströmungen abgedichtet, so dass zwischen dem proximalen Ende der Hülse und dem Kopf keine Absaugverluste entstehen. Der Absaugkanal, der insbesondere auch als Werkzeugkanal ausgebildet sein kann, entfaltet seine Saugwirkung dann am Ende des "festen" Arbeitskanals, also vor Übergang in das Führungselement. Durch die den Kopf umschließende Hülse erstreckt sich die Saugwirkung dann auf die ganze distale offene Stirnseite der Hülse. Der Absaugquerschnitt ist dadurch deutlich vergrößert gegenüber den Querschnitten von herkömmlichen Absaugkanälen, die sich sonst bis zur Stirnseite des Kopfes erstrecken. Der geringere Flächendurchsatz beim Absaugen mit der erfindungsgemäßen Hülse verringert oder verhindert vorteilhaft die Gefahr des unerwünschten Festsaugens an Schleimhäuten oder anderen Gefäßwänden.

Die Hülse kann gleichzeitig auch als Anschlag für das Führungselement ausgebildet sein. Weiterhin kann sie als Befestigungsmittel des Führungselements insoweit dienen, als das Führungselement bei aufgeschobener Hülse nicht von seiner Schwenkachse abgezogen werden kann und somit in seiner gewünschten Montageposition bleibt. Die Demontage des Führungselements ist entsprechend vorteilhaft einfach möglich, in dem zunächst die Hülse abgenommen und dann das Führungselement - im wesentlichen quer zur Längsachse des Endoskops - von seine Schwenkachse abgezogen wird.

Die Hülse kann mit dem Kopf steckbar, schraubbar oder rastbar mittels einer lösbaren oder unlösbaren Verbindung verbunden sein, und so eine einfache Montage oder Demontage ermöglichen.

Eine weitere Ausführungsform der Erfindung sieht vor, dass das Führungselement (F) mit dem Kopf (K) steckbar, schraubbar oder rastbar mittels einer lösbaren oder mittels einer unlösbaren Verbindung verbunden ist. Eine lösbare, also abnehmbare Verbindung erleichtert vorteilhaft die Reinigung oder den Austausch von einzelnen Teilen in diesem Bereich, während eine unlösbare Verbindung zu einer stabilen und robusten Bauform führt.

Weitere vorteilhafte Ausführungsformen ergeben sich aus den Unteransprüchen.

Anhand des nachfolgenden Figurenbeispiels sollen zwei Ausführungsformen der Erfindung beispielhaft erläutert werden. Von den Figuren zeigt.
- Fig. 1: einen Teilschnitt einer ersten Variante des Endoskopkopfs,
- Fig. 2: einen Teilschnitt einer zweiten Ausführungsvariante,
- Fig. 3: eine schematische Darstellung einer weiteren Ausführungsform,
- Fig. 4: die Ausführungsform gemäß Fig. 3 mit eingeführtem Werkzeug.

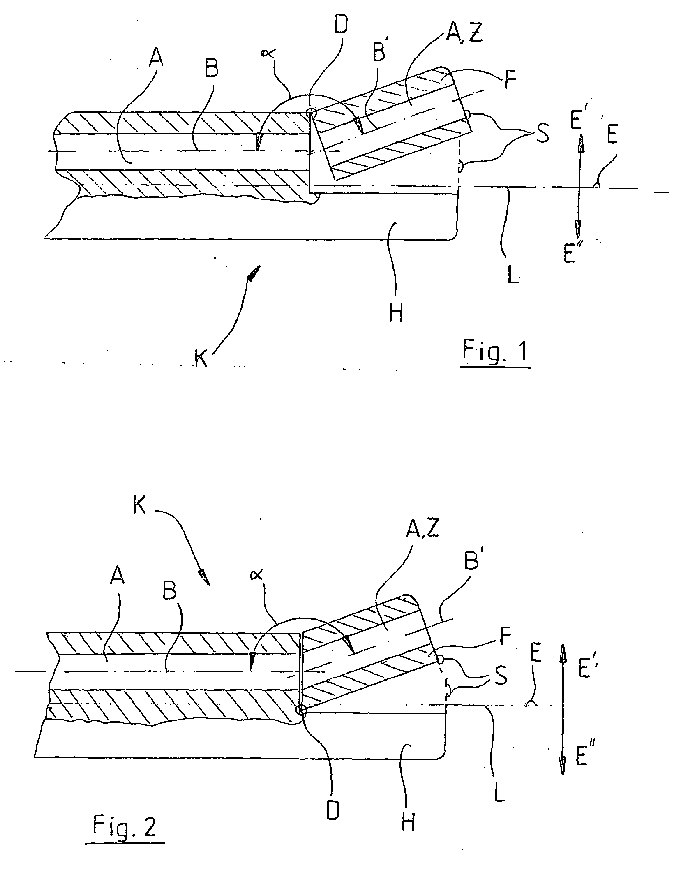

Wie in Fig. 1 zu erkennen ist, ist ein Endoskopkopf K vorgesehen, welcher das distale Ende eines sich in der Zeichnung von links erstreckenden Endoskops darstellt.

Der Kopf K weist eine Stirnseite S auf, die das Endoskop am distalen Ende abschließt. Teil des Kopfes K ist ein starrer Abschnitt H, der sich bis zur Stirnseite S erstreckt. Nicht dargestellt sind verschiedene Kanäle und Einheiten, die sich beispielsweise im starren Abschnitt H bis zur Stirnseite erstrecken können (optische Systeme, Spülkanäle, etc.).

Der Endoskopkopf ist im Wesentlichen entlang der Längsrichtung L des Endoskops ausgerichtet. Vom proximalen Ende her erstreckt sich ein Arbeitskanal A, der im Wesentlichen parallel zur Längsrichtung L ausgerichtet ist. Der Arbeitskanal A verläuft ebenfalls bis zur Stirnseite S des Kopfes K, so dass ein durch den Arbeitskanal A durchgeschobenes Werkzeug in Längsrichtung L aus dem Endoskopkopf heraustreten kann.

In einem ersten Abschnitt des Endoskopkopfs K verläuft der Arbeitskanal A entlang einer ersten Kanallängsachse B.

In Richtung auf die Stirnseite S weist der Endoskopkopf K ein Führungselement F auf. Der Arbeitskanal A wird durch das Führungselement F hindurch weitergeführt, und wird in diesem letzten Abschnitt mit Z bezeichnet. Der letzte Abschnitt Z des Arbeitskanals A verläuft im Führungselement F entlang einer Kanallängsachse B'.

Das Führungselement F ist um eine Schwenkachse D schwenkbar. In Fig. 1 ist die Schwenkachse D senkrecht zur Zeichenebene ausgeführt, und erstreckt sich damit im Wesentlichen auch senkrecht zur in der Zeichnung horizontal ausgeführten Längsrichtung L. Das Führungselement F ist um die Schwenkachse D so schwenkbar, dass zwischen den Kanallängsachsen B und B' ein Winkel α eingeschlossen wird. Im unverschwenkten Zustand ergibt der Winkel α 180°, so dass die Kanallängsrichtungen B und B' fluchten.

Wird das Führungselement F aus der fluchtenden Position nach außen geschwenkt, so dass ein Winkel a < 180° von den beiden Kanallängsachsen B und B' eingeschlossen wird, so wird ein durch den letzten Abschnitt Z durchgeschobenes Werkzeug aus dem Bereich der Längsrichtung L heraus (im gezeichneten Fall nach oben) herausgelenkt. Das Werkzeug erhält damit Zugriff auf einen weiteren Bereich des Operationsorts.

In Fig. 2 ist ein in weiten Teilen identischer Endoskopkopf K dargestellt, bei dem die Schwenkachse D an anderer Stelle angeordnet ist. Das Führungselement F schwenkt dabei um die Schwenkachse D, wobei die Achse im Wesentlichen durch den Endoskopkopf verläuft, im Gegensatz zu einer im Wesentlichen am Rande des Kopfs gelegenen Anordnung gemäß Fig. 1.

Das Führungselement F ist in dem Bereich, in dem der Kanalabschnitt Z in den Kanalabschnitt A übergeht, konstruktiv so abgeschrägt, dass die Schwenkbewegung um ein definiertes Maß möglich ist. Je nach Maß der Abschrägung ist damit auch ein Anschlag definiert, der die Schwenkbewegung des Führungselements F von der Längsrichtung L nach außen begrenzt.

Eine entlang der Längsachse L gedachte Mittelebene E soll dabei verdeutlichen, dass sich das Führungselement F in Richtung (nicht notwendiger Weise bis) auf die eine Seite E' ebenso schwenken lassen soll wie in Richtung (nicht notwendiger Weise bis) auf die andere Seite E".

In Fig. 3 ist eine abgewandelte Ausführungsform eines erfindungsgemäßen Endoskopkopfes dargestellt. Zu erkennen ist ein von links kommender Arbeitskanal A, der sich entlang einer Kanalachse B erstreckt. Der Arbeitskanal A mündet im Kopfbereich des Endoskops in ein Führungselement F, in dessen Innerem der Arbeitskanal A in einem letzten Abschnitt Z fortgeführt wird. Das Führungselement F ist um eine Schwenkachse D schwenkbar.

Ein Betätigungszug C ist vom proximalen Ende des Endoskops kommend seitlich am Führungselement F angelenkt. Der Betätigungszug C ist gegen die Kraft eines Federelements W so verschieblich, dass das Führungselement F um die Schwenkachse D geschwenkt wird.

Das Führungselement F weist dabei an seinem proximalen Ende zwei als Anschlagflächen wirkende Stirnseiten G auf, die einen Winkel β definieren, der gleichzeitig die maximale Schwenkbewegung des Führungselements F um die Schwenkachse D bestimmt. Der Betätigungszug C vermag dabei das Führungselement F so zu bewegen, das es aus einer ersten Anschlagposition (wie sie in Fig. 3 und 4 dargestellt ist) um die Schwenkachse D (in Fig. 3 und 4 gegen den Uhrzeigersinn nach oben) in einen zweite Anschlagposition gelangt, wobei in jeder Anschlagposition eine der Stirnseiten G mit einer Anschlagfläche M des Kopfes zusammenwirkt.

In Fig. 4 ist die gleiche Ausführungsform dargestellt, diesmal jedoch mit einem in den Arbeitskanal A eingeführten Werkzeug. Das Werkzeug erstreckt sich in der vereinfachten Darstellung als Schlauch vom proximalen Ende kommend her bis durch das Führungselement F hindurch und ragt über die Stirnseite S hinaus. Wie zu erkennen ist, wird das Werkzeug insbesondere im Bereich des Führungselementes F von diesem Führungselement F allseitig umschlossen und somit sicher geführt. Das Werkzeug ist in dem Arbeitskanal A bzw. im Führungselement F in Längsrichtung verschieblich und gleichzeitig im Bereich der Stirnseite S durch Verschwenken des Führungselements F um die in Fig. 4 nicht dargestellte Schwenkachse D auf die beiden Seiten E' und E" der Ebene E schwenkbar.

Der Kopf K des Endoskops wird von einer Hülse T umschlossen. Die Hülse T wird auf ihrer dem proximalen Ende des Endoskops zuweisenden Seite durch den Kopf selber so abgedichtet, dass zwischen der Hülse T und dem Kopf K kein Medium hindurchströmen kann.

Auf der dem distalen Ende zugewandten Öffnung der Hülse T ist diese jedoch offen. Der Arbeitskanal A ist gleichzeitig als Saugkanal ausgebildet, so dass durch ihn in proximale Richtung Medium aus dem Kopfbereich des Endoskops abgesaugt werden kann. Der Arbeitskanal A, der gleichzeitig Saugkanal ist, erweitert sich beim Übergang in das Führungselement F bis auf den Durchmesser der Hülse T. Auf diese Weise kann über den gesamten von der Hülse T am distalen Ende gebildeten Querschnitt abgesaugt werden. Die Hülse T ist dabei mit dem Endoskopkopf K beispielsweise verschraubt oder steckbar verbunden.

## Patentansprüche

1. Flexibles Endoskop mit einer Längsrichtung (L), mit einem proximalen und einem distalen Ende und einem am distalen Ende angeordneten Endoskopkopf (K), wobei
a) der Endoskopkopf (K) eine das Endoskop im Wesentlichen distal begrenzende und zur Längsrichtung (L) lotrecht gedachte Stirnseite (S) aufweist, und wobei
b) das Endoskop wenigstens einen sich in Richtung auf die Stirnseite (S) erstreckenden und im Wesentlichen parallel zur Längsrichtung (L) entlang einer Kanallängsachse (B) verlaufenden Arbeitskanal (A) zur Führung von Werkzeugen und/oder Bedienungselementen und zur Durchströmung von Medien aufweist, und wobei
c) eine in der Längsrichtung (L) liegende Ebene (E) eine erste Seite (E') und eine dieser Seite (E') abgewandte zweite Seite (E") aufweist, und wobei
d) der Kopf (K) ein den letzten Abschnitt (Z) des Kanals (A) bildendes Führungselement (F) aufweist,
d1) in welchem der Arbeitskanal (A,Z) entlang einer Kanallängsachse (B') ausgebildet ist, und
d2) welches relativ zum Kopf (K) um eine Schwenkachse (D) zur ersten Seite (E') und/oder zur zweiten Seite (E") hin so schwenkbar ist, dass die Kanallängsachse (B) des Endoskops und die Kanallängsachse (B') des Führungselements einen Winkel α ≠ 180° bilden, wobei
e) die proximale Begrenzung des Führungselements (F) zwei einen Winkel β < 180° bildende Stirnflächen (G) aufweist, in deren gedachtern Schnittbereich die Schwenkachse (D) so angeordnet ist, dass die Stirnflächen als die Schwenkbewegung begrenzende, mit Anschlagabschnitten (M) des Kopfes (K) zusammenwirkende Anschlagflächen ausgebildet sind, und wobei
f) das Endoskop eine sich in Längsrichtung (L) erstreckende, den Kopf (K) radial umschließende und in distaler Richtung offene Hülse (T) aufweist, und
g) wobei das distale offene Ende der Hülse (T) eine Absaugöffnung des Endoskops bildet,
**dadurch gekennzeichnet, dass**
h) das Führungselement (F) zur genauen Führung eines Werkzeugs dieses knapp umschließt und zur Aufrechterhaltung einer Saugwirkung mit Ausnehmungen, insbesondere mit in axialer Richtung verlaufenden Vertiefungen ausgebildet ist.

2. Endoskop nach Anspruch 1, **dadurch gekennzeichnet, dass** die Schwenkachse (D) quer zur Längsrichtung (L) angeordnet ist.

3. Endoskop nach einem der vorigen Ansprüche, **dadurch gekennzeichnet, dass** die Schwenkbewegung des Führungselements (F) durch wenigstens einen Anschlag in wenigstens einer Schwenkrichtung begrenzt wird.

4. Endoskop nach Anspruch 3, **dadurch gekennzeichnet, dass** der von der Hülse (T) umschlossene Raum in proximaler Richtung durch den Kopf (K) abgedichtet ist und/oder als Anschlag für das Führungselement (F) ausgebildet ist.

5. Endoskop nach Anspruch 3 oder 4, **dadurch gekennzeichnet, dass** die Hülse (T) mit dem Kopf (K) steckbar, schraubbar oder rastbar mittels einer lösbaren oder unlösbaren Verbindung verbunden ist.

6. Endoskop nach einem der vorigen Ansprüche, **dadurch gekennzeichnet, dass** das Führungselement (F) mit dem Kopf (K) steckbar, schraubbar oder rastbar mittels einer lösbaren oder mittels einer unlösbaren Verbindung verbunden ist.

7. Endoskop nach einem der vorigen Ansprüche, **dadurch gekennzeichnet, dass** das Führungselement (F) gegen die Kraft eines Federelements (W) mittels eines Betätigungszuges (C) schwenkbar ist, wobei das Federelement (W) das Führungselement (F) im unbetätigten Zustand in eine vorgebbare Lage schwenkt.

8. Endoskop nach einem der vorigen Ansprüche, **dadurch gekennzeichnet, dass** ein das Führungselement (F) bewegendes Betätigungselement zu Reinigungs- und Wartungszwecken aus dem Endoskop entnehmbar ist.

## Claims

1. Flexible endoscope having a longitudinal direction (L), and having a proximal and a distal end and an endoscope head (K) arranged at the distal end, wherein
a) the endoscope head (K) has a notional front end (S) which substantially delimits the endoscope distally and is perpendicular to the longitudinal direction (L), and wherein
b) the endoscope has at least one working channel (A), for guiding tools and/or operating elements and for the flow-through of media, which working channel extends in the direction of the front end (S) and runs along a channel longitudinal axis (B) substantially parallelwise relative to the longitudinal direction (L), and wherein
c) a plane (E) lying in the longitudinal direction (L) has a first side (E') and a second side (E") which faces away from this side (E'), and wherein
d) the head (K) has a guide element (F) which constitutes the final portion (Z) of the channel (A),
d1) in which the working channel (A,Z) is realized along a channel longitudinal axis (B'), and
d2) which is pivotable, relative to the head (K), about a pivot axis (D) towards the first side (E') and/or towards the second side (E") in such a way that the channel longitudinal axis (B) of the endoscope and the channel longitudinal axis (B') of the guide element form an angle α # 180°, wherein
e) the proximal delimitation of the guide element (F) has two end faces (G) which form an angle β < 180° and in whose notional intersection region the pivot axis (D) is so arranged that the end faces are realized as stop faces which delimit the pivoting movement and which cooperate with stop portions (M) of the head (K), and wherein
f) the endoscope has a sleeve (T) which extends in the longitudinal direction (L), encompasses the head (K) radially and is open in the distal direction, and
g) wherein the distal, open end of the sleeve (T) constitutes a suction extraction aperture of the endoscope,
**characterized in that**
h) the guide element (F), for the purpose of accurately guiding a tool, encompasses the latter closely and, for the purpose of maintaining a suction action, is realized with recesses, in particular with hollows running in the axial direction.

2. Endoscope according to Claim 1, **characterized in that** the pivot axis (D) is arranged transversely relative to the longitudinal direction (L).

3. Endoscope according to either one of the preceding claims, **characterized in that** the pivoting movement of the guide element (F) is delimited in at least one pivoting direction by at least one stop.

4. Endoscope according to Claim 3, **characterized in that** the space encompassed by the sleeve (T) is sealed in the proximal direction by the head (K) and/or is realized as a stop for the guide element (F).

5. Endoscope according to either of Claims 3 or 4, **characterized in that** the sleeve (T) is connected to the head (K) such that it can be pushed on, screw-connected or latched by means of a disconnectable or non-disconnectable connection.

6. Endoscope according to any one of the preceding claims, **characterized in that** the guide element (F) is connected to the head (K), such that it can be pushed on, screw-connected or latched by means of a disconnectable connection, or by means of a non-disconnectable connection.

7. Endoscope according to any one of the preceding claims, **characterized in that** the guide element (F) is pivotable against the force of a spring element (W) by means of an actuating pull (C), the spring element (W) pivoting the guide element (F), in the non-actuated state, into a predefinable position.

8. Endoscope according to any one of the preceding claims, **characterized in that** an actuating element which moves the guide element (F) is removable from the endoscope for the purpose of cleaning and servicing.

## Revendications

1. Endoscope souple pourvu d'une direction longitudinale (L), d'une extrémité proximale et d'une extrémité distale et d'une tête d'endoscope (K) agencée à l'extrémité distale (K), dans lequel
a) la tête d'endoscope (K) présente un côté frontal (S) délimitant l'endoscope de manière sensiblement distale et élaboré perpendiculairement à la direction longitudinale (L), dans lequel
b) l'endoscope présente au moins un canal de travail (A) s'étendant en direction du côté frontal (S) et progressant sensiblement parallèlement à la direction longitudinale (L) le long d'un axe longitudinal (B) du canal pour guider des outils et/ou des éléments de commande et pour le passage de substances, dans lequel
c) un plan (E) situé dans la direction longitudinale (L) présente un premier côté (E') et un second côté (E") opposé à ce côté (E') et dans lequel
d) la tête (K) présente un élément de guidage (F) formant le dernier segment (Z) du canal (A),
d1) dans lequel le canal de travail (A,Z) est formé le long d'un axe longitudinal (B') du canal, et
d2) qui peut pivoter par rapport à la tête (K) autour d'un axe de pivotement (D) vers le premier côté (E') et/ou vers le second côté (E") de sorte que l'axe longitudinal (B) du canal de l'endoscope et l'axe longitudinal (B') du canal de l'élément de guidage forment un angle α ≠ 180°, dans lequel
e) la délimitation proximale de l'élément de guidage (F) présente deux surfaces frontales (G) formant un angle β < 180°, dans la zone d'intersection élaborée desquelles est agencé l'axe de pivotement (D) de sorte que les surfaces frontales se présentent sous forme de surfaces de butée limitant le mouvement pivotant et coopérant avec des segments de butée (M) de la tête (K) et dans lequel
f) l'endoscope présente une gaine (T) s'étendant dans la direction longitudinale (L), enserrant radialement la tête (K) et ouverte dans la direction distale, et
g) dans lequel l'extrémité distale ouverte de la gaine (T) forme une ouverture d'aspiration de l'endoscope,
**caractérisé en ce que**
h) l'élément de guidage (F) enserre étroitement un instrument pour le guider de manière précise et présente, pour conserver un effet d'aspiration, des évidements, en particulier des cavités s'étendant dans la direction axiale.

2. Endoscope selon la revendication 1, **caractérisé en ce que** l'axe de pivotement (D) est agencé transversalement à la direction longitudinale (L).

3. Endoscope selon l'une quelconque des revendications précédentes, **caractérisé en ce que** le mouvement pivotant de l'élément de guidage (F) est délimité par au moins une butée dans au moins une direction de pivotement.

4. Endoscope selon la revendication 3, **caractérisé en ce que** l'espace enserré par la gaine (T) est étanché dans la direction proximale par la tête (K) et/ou se présente sous la forme d'une butée pour l'élément de guidage (F).

5. Endoscope selon la revendication 3 ou 4, **caractérisé en ce que** la gaine (T) peut être raccordée à la tête (K) par enfichage, vissage ou encliquetage à l'aide d'un raccordement enlevable ou non.

6. Endoscope selon l'une quelconque des revendications précédentes, **caractérisé en ce que** l'élément de guidage (F) peut être raccordé à la tête (K) par enfichage, vissage ou encliquetage à l'aide d'un raccordement enlevable ou non.

7. Endoscope selon l'une quelconque des revendications précédentes, **caractérisé en ce que** l'élément de guidage (F) peut pivoter à l'encontre de la force d'un élément à ressort (W) à l'aide d'une tirette de commande (C) de sorte que l'élément à ressort (W) fasse pivoter l'élément de guidage (F) à l'état non commandé dans une position prédéfinissable.

8. Endoscope selon l'une quelconque des revendications précédentes, **caractérisé en ce qu'**un élément de commande déplaçant élément de guidage (F) peut être retiré de l'endoscope à des fins de nettoyage et d'entretien.
